# EUROPEAN PATENT APPLICATION

(11) **EP 2 039 780 A1**
(43) Date of publication of application: **25.03.2009**
(21) Application number: 07018755.4
(22) Date of filing: 24.09.2007
(51) Int. Cl.: C12Q 1/68

(54) **Single-readout multiplexing of metagenes**

(71) Applicant: Siemens Healthcare Diagnostics GmbH, 65760 Eschborn (DE)
(72) Inventor: Feder, Inke Sabine, 51377 Leverkusen (DE); Kronenwett, Ralf, Dr., 40237 Düsseldorf (DE); Stropp, Udo, Dr., 42781 Haan (DE)
(74) Representative: Maier, Daniel Oliver

(57) **Abstract**

The present invention is related to a method of analyzing a biological sample comprising the following steps: (a) providing a first group of probes comprising at least a first probe capable of specifically binding a first biological molecule belonging to a first group of biological molecules and a second probe capable of specifically binding a second biological different from the first biological molecule, but also belonging to the first group of biological molecules, (b) reacting said first probe and said second probe with said sample such as to form a binding complex of the probes with the respective biological molecule, (c) providing a first label yielding a detectable signal upon binding of the first probe to the first biological molecule or upon binding of the second probe to the second biological molecule, wherein a combined detectable first signal results when one or both of first and second biological molecule are present in the sample; and (d) reacting said first label to detect binding of probes with the respective biological molecule. The biological molecules may be gene products of housekeeping genes in order to provide improved internal controls for gene expression analysis assays.

SEQUENCE LISTING

<110> Siemens Medical Solutions Diagnostics Gmbh

<120> Single-Readout Multiplexing of Metagenes

<130> 200715205

<160> 24

<170> PatentIn version 3.3

<210> 1
<211> 20
<212> DNA
<213> Artificial

<220>
<223> PCR Primer, Probe or Product

<400> 1
aggcccccct gaaccccaag 20

<210> 2
<211> 24
<212> DNA
<213> Artificial

<220>
<223> PCR Primer, Probe or Product

<400> 2
tggttggatg gcaagcatgt ggtg 24

<210> 3
<211> 22
<212> DNA
<213> Artificial

<220>
<223> PCR Primer, Probe or Product

<400> 3
tcgcgtctcg gaaaccggta gc 22

<210> 4
<211> 23
<212> DNA
<213> Artificial

<220>
<223> PCR Primer, Probe or Product

<400> 4
tgcttccaca agaaccgcga gga 23

<210>  5
<211>  28
<212>  DNA
<213>  Artificial

<220>
<223>  PCR Primer, Probe or Product

<400>  5
aaggtgaagg tcggagtcaa cggatttg                                          28


<210>  6
<211>  18
<212>  DNA
<213>  Artificial

<220>
<223>  PCR Primer, Probe or Product

<400>  6
tggctggcgg tgcctgga                                                     18


<210>  7
<211>  22
<212>  DNA
<213>  Artificial

<220>
<223>  PCR Primer, Probe or Product

<400>  7
ctggcaccac accttctaca ac                                                22


<210>  8
<211>  22
<212>  DNA
<213>  Artificial

<220>
<223>  PCR Primer, Probe or Product

<400>  8
tttcatctgc actgccaaga ct                                                22


<210>  9
<211>  20
<212>  DNA
<213>  Artificial

<220>
<223>  PCR Primer, Probe or Product

<400>  9
gagcgagctg agtggttgtg                                                   20

<210>  10
<211>  20
<212>  DNA
<213>  Artificial

<220>
<223>  PCR Primer, Probe or Product

<400>  10
cgagccgacc atgtcttcat                                                    20


<210>  11
<211>  17
<212>  DNA
<213>  Artificial

<220>
<223>  PCR Primer, Probe or Product

<400>  11
gccagccgag ccacatc                                                       17


<210>  12
<211>  21
<212>  DNA
<213>  Artificial

<220>
<223>  PCR Primer, Probe or Product

<400>  12
tgtggttcct gcatgaagac a                                                  21


<210>  13
<211>  30
<212>  DNA
<213>  Artificial

<220>
<223>  PCR Primer, Probe or Product

<400>  13
caaacataat ctgagtcatc ttctctctgt                                         30


<210>  14
<211>  24
<212>  DNA
<213>  Artificial

<220>
<223>  PCR Primer, Probe or Product

<400>  14
tattcatgcc ttctttcact ttgc                                               24

<210>  15
<211>  21
<212>  DNA
<213>  Artificial

<220>
<223>  PCR Primer, Probe or Product

<400>  15
agtcagttgg tcagccatgc t                                                21


<210>  16
<211>  21
<212>  DNA
<213>  Artificial

<220>
<223>  PCR Primer, Probe or Product

<400>  16
aagcccaaaa agctgaaggt t                                                21


<210>  17
<211>  16
<212>  DNA
<213>  Artificial

<220>
<223>  PCR Primer, Probe or Product

<400>  17
ccaggcgccc aatacg                                                      16


<210>  18
<211>  24
<212>  DNA
<213>  Artificial

<220>
<223>  PCR Primer, Probe or Product

<400>  18
gtgacagcgg aagtggtatt gtac                                             24


<210>  19
<211>  119
<212>  DNA
<213>  Artificial

<220>
<223>  PCR Primer, Probe or Product

<400>  19
ctggcaccac accttctaca acgagctgcg cgtggccccg gaggagcacc cagtgctgct      60

gaccgaggcc cccctgaacc ccaaggccaa cagagagaag atgactcaga ttatgtttg      119

<210>  20
<211>  77
<212>  DNA
<213>  Artificial

<220>
<223>  PCR Primer, Probe or Product

<400>  20
tttcatctgc actgccaaga ctgagtggtt ggatggcaag catgtggtgt ttggcaaagt      60

gaaagaaggc atgaata                                                     77


<210>  21
<211>  72
<212>  DNA
<213>  Artificial

<220>
<223>  PCR Primer, Probe or Product

<400>  21
gagcgagctg agtggttgtg tggtcgcgtc tcggaaaccg gtagcgcttg cagcatggct      60

gaccaactga ct                                                          72


<210>  22
<211>  83
<212>  DNA
<213>  Artificial

<220>
<223>  PCR Primer, Probe or Product

<400>  22
cgagccgacc atgtcttcat ttgcttccac aagaaccgcg aggacagagc cgccttgctc      60

cgaaccttca gctttttggg ctt                                              83


<210>  23
<211>  80
<212>  DNA
<213>  Artificial

<220>
<223>  PCR Primer, Probe or Product

<400>  23
gccagccgag ccacatcgct cagacaccat ggggaaggtg aaggtcggag tcaacggatt      60

tggtcgtatt gggcgcctgg                                                  80


<210>  24
<211>  65

<212>  DNA
<213>  Artificial

<220>
<223>  PCR Primer, Probe or Product

<400>  24
tgtggttcct gcatgaagac agtggctggc ggtgcctgga cgtacaatac cacttccgct      60

gtcac                                                                  65

## Description

The present invention relates to the analysis of biological molecules in a biological sample and a kit for performing that method. More specifically, the present invention relates to a single-readout analysis of multiple genes, can also known as multiplexing.

Technologies such as microarray analysis, quantitative PCR and others allow the analysis of genome-wide expression pattern which provides new insights into gene regulation and is also a useful diagnostic tool, because it allows the analysis of pathologic conditions at the level of gene expression. Quantitative reverse transcriptase PCR is currently the accepted standard for quantifying gene expression. It has the advantage of being a very sensitive method allowing the detection of even minute amounts of mRNA. Microarray analysis is fast becoming a new standard for quantifying gene expression. A microarray is an array of addressable positions, wherein each position carries a specific probe for detecting a biological molecule. Most commonly, a microarray is provided as a so called bio chip, wherein e.g. DNA probes are immobilized on the chip surface. Both methods can be adapted to automated procedures for generating high throughput performance and play an increasingly important role in research and diagnostics. For example, many tumors are characterized by a specific gene expression pattern and the analysis of this pattern yields information of diagnostic, prognostic and predictive value. A common problem in this type of analysis is the need to compare the expression level of genes against a known control or standard. This is necessary to compare a given sample with another sample which can for example be a reference sample from the same patient at a different time point A large number of different assay systems, for example, quantitative PCR (qPCR), microarray, northern blot analysis etc., use so called housekeeping genes as internal controls against which the expression level of a gene of interest (GOI) is compared. Housekeeping genes are control genes, which are selected because of their stable and constant expression in a wide variety of tissues or cells.

Many housekeeping genes are vital to the metabolism of viable cells and are therefore constantly expressed. Many housekeeping genes encode enzymes or structural RNAs, such as ribosomal RNAs, which perform essential metabolic functions (hence the name "housekeeping") and are therefore constantly expressed. However, it has been found that expression of these genes may vary considerably, especially in tumor cells, and their expression level may also vary over time. This is especially problematic in the analysis of tumor cells with aberrant gene expression and carries resulting implications for research and diagnostic purposes. See also Lee et al, Journal of Biochemistry and Molecular Biology, Vol. 40, No. 2, March 2007, pp. 226-231; Silver et al., BMC Molecular Biology 2006, Vol. 7, p. 33-40. Table 1 lists some exemplary housekeeping genes. The prerequisite of a suitable housekeeping gene is that it should, of course, be adequately expressed in the tissue of interest, but most importantly, that it shows minimal variability in expression between samples and under the experimental conditions used. It is still common practice in most of the RNA transcription analysis published today that a single housekeeping gene is used as internal control. However, it is quite clear that many of these control genes show unacceptable variability in expression. Appropriate validation of housekeeping genes in any new experimental system is therefore crucial, but also time consuming and requires valuable experimental resources.

To solve this problem it has been proposed in the prior art to analyze a large number of housekeeping genes with regard to their expression level and select those housekeeping genes as control which for a given purpose (e.g. analysis of a particular type of tumor) provide the most constant expression level. Various attempts to find the most appropriate method for the selection of housekeeping genes have been made. For example, a software-based approach, developed by Vandesompele et al involves the use of normalizing to more than one housekeeping gene, where, very briefly, an algorithm based computer program is used to determine the most stable control genes from a panel of candidate housekeeping genes via a stepwise exclusion or ranking process, and this is then followed by geometric averaging of a selection of the most stable control genes, (Vandesompele et al., Genome Biol. 2002 Jun 18;3(7):RESEARCH0034. Epub 2002 Jun 18). This, however, still requires separate measurements for a plurality of housekeeping gene to be made and then mathematically averaging the expression levels.

**Table 1: Exemplary housekeeping genes (from: Vandesompele et al.)**

| Symbol | Accession Number, Gene Title | | Function |
|---|---|---|---|
| *ACTB* | NM_001101 | Beta actin | Cytoskeletal structural protein |
| *B2M* | NM_004048 | Beta-2-microglobulin | Beta-chain of major histocompatibility complex class I molecules |
| *GAPD* | NM_002046 | Glyceraldehyde-3-phosphate dehydrogenase | Oxidoreductase in glycolysis and gluconeogenesis |
| *HMBS* | NM_000190 | Hydroxymethyl-bilane synthase | Heme synthesis, porphyrin metabolism |
| *HPRT1* | NM_000194 | Hypoxanthine phosphoribosyl-transferase 1 | Purine synthesis in salvage pathway |
| *RPL 13A* | NM_012423 | Ribosomal protein L13a | Structural component of the large 60S ribosomal subunit |
| *SDHA* | NM_004168 | Succinate dehydrogenase complex, subunit A | Electron transporter in the TCA cycle and respiratory chain |
| *TBP* | NM_003194 | TATA box binding protein | General RNA polymerase II transcription factor |
| *UBC* | M26880 | Ubiquitin C | Protein degradation |
| *YWHAZ* | NM_003406 | Tyrosine 3-monooxygenase/ tryptophan 5-monooxygenase binding to phosphorylated activation protein, zeta polypeptide | Signal transduction |

In qPCR studies it is common to measure up to six or more housekeeping genes in parallel to the gene of interest. A CT value (cycle threshold of exponential growth in qPCR) is used as quantifiable signal and gene expression is determined using the mean of expression levels of the housekeeping genes (normalization standard) and the expression levels of a gene of interest. This type of normalization or control is required, because several variables need to be controlled for gene expression analysis, e.g. the amount of starting material, the quality of RNA, differences between tissues or cells in overall transcriptional activity and so on. The level of gene expression of a gene of interest (GOI) may be then expressed relative to the expression of the control (i.e. housekeeping gene), e.g. as delta CT = CT[GOI] - CT[control].

Other solutions to this problem have included measuring total RNA mass quantity of a given sample, but this does not take in to account the quality of RNA or differences in enzymatic activity, for example, when a reverse transcription step is included in the analysis. Further, a variation in the expression level of control genes reduces overall sensitivity, as small differences in expression levels become impossible to detect.

The currently used method of measuring the expression level of a plurality of housekeeping genes and then mathematically forming an average requires several single reactions (one for each gene or mRNA-species) and thus requires valuable resources, e.g. additional enzymes, labels or valuable microarray-space.

### Objective of the invention

It is thus an objective of the present invention to provide an improved internal control for gene expression assays or studies.

It is a further objective of the invention to provide a screening method for the analysis of metagenes.

### Definitions

The term "metagenes" within the meaning of the present invention refers to a group of genes commonly linked to some phenotype, common pathological condition, or which as a group share some common functions, for example, housekeeping genes which maybe used as internal controls for gene expression assays or studies. Often metagenes have some mechanism of gene regulation in common which may lead to a common gene expression pattern, e.g. constant level of expression, disease-dependent overexpression, disease-dependent absence of expression, and others.

In the context of the present invention a "biological sample" is a sample which is derived from or has been in contact with a biological organism. Examples for biological samples are: cells, tissue, body fluids, biopsy specimens, blood, urine, saliva, sputum, plasma, serum, cell culture supernatant, and others.

A "biological molecule" within the meaning of the present invention is a molecule generated or produced by a biological organism or indirectly derived from a molecule generated by a biological organism, such as: nucleic acids, protein, polypeptide, peptide, DNA, mRNA, cDNA, and so on.

A "probe" is a molecule or substance capable of specifically binding or interacting with a specific biological molecule. The term "primer", "primer pair" or "probe", shall have ordinary meaning of these terms which is known to the person skilled in the art of molecular biology. In a preferred embodiment of the invention "primer", "primer pair" and "probes" refer to oligonucleotide or polynucleotide molecules with a sequence identical to, complementary too, homologues of, or homologous to regions of the target molecule or target sequence which is to be detected or quantified, such that the primer, primer pair or probe can specifically bind to the target molecule, e.g. target nucleic acid, gene, transcript, peptide, polypeptide, or protein to be detected or quantified. As understood herein, a primer may in itself function as a probe. A "probe" as understood herein may also comprise e.g. a combination of primer pair and internal labeled probe, as is common in many commercially available qPCR methods.

A "gene" is a set of segments of nucleic acid that contains the information necessary to produce a functional RNA product in a controlled manner. A "gene product" is a biological molecule produced through transcription or expression of a gene, e.g. an mRNA or the translated protein.

The term "specifically binding" within the context of the present invention means a specific interaction between a probe and a biological molecule leading to a binding complex of probe and biological molecule, such as DNA-DNA binding, RNA-DNA binding, RNA-RNA binding, DNA-protein binding, protein-protein binding, RNA-protein binding, antibody-antigen binding, and so on.

The term "expression level" refers to a determined level of gene expression. This may be a determined level of gene expression compared to a reference gene (e.g. a housekeeping gene) or to a computed average expression value (e.g. in DNA chip analysis) or to another informative gene without the use of a reference sample. The expression level of a gene may be measured directly, e.g. by obtaining a signal wherein the signal strength is correlated to the amount of mRNA transcripts of that genes or it may be obtained indirectly at a protein level, e.g. by immunohistochemistry, CISH, ELISA or RIA methods. The expression level may also be obtained by way of a competitive reaction to a reference sample.

The term "complementary" or "sufficiently complementary" means a degree of complementarity which is - under given assay conditions - sufficient to allow the formation of a binding complex of a primer or probe to a target molecule. Assay conditions which have an influence of binding of probe to target include temperature, solution conditions, such as composition, pH, ion concentrations, etc. as is known to the skilled person.

The term "hybridization based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are used in order to find - complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. Hybridization is dependent on target and probe (e.g. length of matching sequence, GC content) and hybridization conditions (temperature, solvent, pH, ion concentrations, presence of denaturing agents, etc.).

The term "array" refers to an arrangement of addressable locations on a device, e.g. a chip device. The number of locations can range from several to at least hundreds or thousands. Each location represents an independent reaction site. Arrays include, but are not limited to nucleic acid arrays, protein arrays and antibody-arrays. A "nucleic acid array" refers to an array containing nucleic acid probes, such as oligonucleotides, polynucleotides or larger portions of genes. The nucleic acid on the array is preferably single stranded. A "microarray" refers to a biochip or biological chip, i.e. an array of regions having a density of discrete regions with immobilized probes of at least about 100/cm^{2.}

A "PCR-based method" refers to methods comprising a polymerase chain reaction PCR. This is a method of exponentially amplifying nucleic acids, e.g. DNA or RNA by enzymatic replication in vitro using one, two or more primers. For RNA amplification, a reverse transcription may be used as a first step. PCR-based methods comprise kinetic or quantitative PCR (qPCR) which is particularly suited for the analysis of expression levels,).

The term "determining a protein level" refers to any method suitable for quantifying the amount, amount relative to a standard or concentration of a given protein in a sample. Commonly used methods to determine the amount of a given protein are e.g. immunohistochemistry, CISH, ELISA or RIA methods. etc.

The term "reacting" a probe with a biological molecule to form a binding complex herein means bringing probe and biologically molecule into contact, for example, in liquid solution, for a time period and under conditions sufficient to form a binding complex.

The term "label" within the context of the present invention refers to any means which can yield or generate or lead to a detectable signal when a probe specifically binds a biological molecule to form a binding complex. This can be a label in the traditional sense, such as enzymatic label, fluorophore, chromophore, dye, radioactive label, luminescent label, gold label, and others. In a more general sense the term "label" herein is meant to encompass any means capable of detecting a binding complex and yielding a detectable signal, which can be detected, e.g. by sensors with optical detection, electrical detection, chemical detection, gravimetric detection (i.e. detecting a change in mass), and others. Further examples for labels specifically include labels commonly used in qPCR methods, such as the commonly used dyes FAM, VIC, TET, HEX, JOE, Texas Red, Yakima Yellow, quenchers like TAMRA, minor groove binder, dark quencher, and others or probe indirect staining of PCR products by for example SYBR Green. Readout can be performed on hybridization platforms, like Affymetrix, Agilent, Illumina, Planar Wave Guides, Luminex, microarray devices with optical, magnetic, electrochemical, gravimetric detection systems, and others.

The term "combined detectable signal" within the meaning of the present invention means a signal, which results, when at least two different biological molecules form a binding complex with their respective probes and one common label yields a detectable signal for either binding event.

### Summary of the invention

The present invention is predicated on the fact that joint detection of metagenes in a single reaction, in particular in a single vessel reaction, provides at least comparable or superior results to multiple single analysis of each gene of a group of metagenes while at the same time being more reliable, efficient and economical. This principle can further be used to screen a sample for the presence of any one of a group of genes or for the expression of any one of a group of genes by simultaneously analyzing the group of genes jointly in one reaction to yield a combined detectable signal.

Specifically, the method of the present invention comprises the following steps:
- providing a first group of probes comprising at least a first probe capable of specifically binding a first biological molecule belonging to a first group of biological molecules and a second probe capable of specifically binding a second biological molecule different from the first biological molecule, but also belonging to the first group of biological molecules,
- reacting said first group of probes with the sample such as to enable formation of a binding complex of probes with the respective biological molecules,
- providing a first label yielding a detectable first signal upon binding of the first probe to the first biological molecule or upon binding of the second probe to the second biological molecule, wherein a combined detectable first signal results when one or both of first and second biological molecules are present in the sample, and
- detecting said first signal to detect binding of probes with the respective biological molecules.

A "group of biological molecules" may represent a group of metagenes as defined above. According to the method of the present invention, the first biological molecule and the second biological molecule different from the first biological molecule may be bound by a first probe and a second probe different from the first probe, respectively, but may in the alternative be bound also by a common probe capable of binding the first biological molecule and also the second biological molecule different from the first biological molecule, if the probe uses a common binding motive common to both biological molecules yet still allowing a specific binding, i.e. other biological molecules, not sharing the common binding motive are not bound specifically.

The first group of probes may comprise three or more differing probes for respective three or more differing biological molecules resulting in a combined detectable first signal for three or more different biological molecules.

According to a further aspect of the present invention a second group of probes different from the first group of probes and selected to be capable of binding biological molecules belonging to a second group of biological molecules is provided and a second label yielding a second signal different from the first signal is provided, wherein the second signal results upon binding of one or more of the probes of the second group of probes to one or more corresponding biological molecules of the second group of biological molecules.

In this way, for example, two different groups of metagenes can be analyzed and compared, for example, a group of housekeeping genes vs. a group of genes expected to be highly expressed in a pathological condition. According to a further aspect of the present invention a further probe specific to a further biological molecule may be provided and a further label yielding a further signal different from the signal of the first label and second label maybe provided, wherein the further signal results upon binding of the further probe to the further biological molecule. In this way, for example, a further individual gene can be analyzed with respect to, for example, its expression level, and this result can be viewed in light of the data obtained from the analysis of the first group of biological molecules and/or the second group of biological molecules.

According to a further aspect of the present invention at least one of the first, second and further signal may be quantifiably detected. Quantifiable detection is performed in such a way that the resulting signal strength or signal quantification is correlated with the amount or concentration of biological molecule to be analyzed. A quantifiable detection of a signal may result from a direct correlation of the signal to the amount of detected binding complex, for example in optical detection methods were a higher signal intensity or signal strength corresponds to a higher concentration of detected binding complex, or it maybe a more indirect correlation, for example, a cycle threshold of exponential growth as commonly used in qPCR. The signal quantification of the further signal can be compared to the signal quantification of first or second signal.

The signal strength or signal quantification may be correlated to a level of gene expression.

According to an aspect of the present invention the expression level may be determined by one or more of the following methods:
a) a hybridization based method;
b) a PCR based method;
c) determining the protein level; and
d) an array based method.

The signal strength of the further signal (corresponding to the expression of a gene of interest) can be compared to the signal strength of first or second signal. The level of gene expression of a gene of interest (GOI) may be expressed relative to the expression of the control (i.e. housekeeping gene), e.g. as delta CT = CT[GOI] - CT[control] or as difference or quotient of signal strength.

For example, analysis of the expression level of a further biological molecule, such as an individual gene of interest may be compared to the signal strength of the first signal resulting from the combined detectable first signal, which results, when binding complexes of probes of the first group of probes with biological molecules of the first group of biological molecules are performed. In this way, the expression level of a single gene of interest which is disease correlated may be compared to the combined detectable signal correlated to the expression of a group of housekeeping genes.

According to a further aspect of the present invention a method is provided for the analysis of gene expression wherein the first and second biological molecule belonging to the first group of biological molecules consists of mRNA-molecules and the further biological molecules is an mRNA-molecule wherein the signal strength of the further signal is compared to the signal strength of the first signal.

The biological molecules may be gene products of housekeeping genes in order to provide improved internal controls for gene expression analysis assays. According to a further aspect of the present invention, the first group of biological molecules comprises mRNA of housekeeping genes.

In particular, the first group of biological molecules may comprise mRNA of housekeeping genes and the second group of biological molecules or the further biological molecule is linked to a specific phenotype or pathologic condition.

According to a further aspect of the present invention the first group (and/or second group) of biological molecules may comprise biological molecules linked to a common phenotype or pathologic condition wherein threshold signal strength for the first signal is determined and the signal strength above said threshold signal indicates the presence of said phenotype or pathologic condition. In this way, the present invention can be used as a tool for screening large numbers of samples, wherein the combined detectable first signal indicates the presence of any one of the first group of biological molecules, or in other words a possible screening result. If desired, the presence or absence or amount of each individual member of the first group of biological molecules may then be determined individually. This type of analysis is particularly useful for analyzing a group of genes which are linked to some common pathologic condition. It is known for example, that blood clotting defects maybe due to a number of different genetic defects; these genes thus form a group of metagenes within the meaning of the present invention and maybe tested in combination according to the method of the present of invention, wherein a positive first signal indicates the presence of a genetic clotting defect, and this individual sample then maybe analyzed further in a subsequent step. This type of procedure saves time and money, because for screening purposes it is not necessary to measure all single genes individually. This is important when screening thousands of people, for example. When positive results occur each single gene may then be tested afterwards more specifically. According to a related aspect of the present invention, there is provided a method of screening samples such as described above, wherein the first group of biological molecules comprise biological molecules affected by a mutation and wherein the first group of probes is selected such that each probe will bind a respective corresponding molecule if said mutation is present. Examples for -mutation are SNP (Single Nucleotide Polymorphism), deletion, insertion, duplication, amplification, translocation, substitutions, and others. The probes used in the method of the present invention maybe oligonucleotides.

The first signal maybe quantifiably determined by quantitative PCR. This method can be run on any quantitative PCR system. Commercially available systems may be used, for example, "Real Time PCR System" of Applied Biosystems, LightCycler® from Roche, iCycler® from BioRAd, Mx from Stratagene, and others.

According to another aspect of the present invention the probes are provided on a microarray and the first probe and second probe of the first group of probes are provided at the same array-position. This results in a combined detectable signal at that array-position when any one of the biological molecules of the first group of biological molecules is present.

According to a further aspect of the present invention at least one of the probes is an oligonucleotide comprising a sequence selected from the group consisting of SEQ ID NO:1 to SEQ ID NO:18.

According to a further aspect of the present invention, the first probe and second probe of the first group of probes maybe provided at different concentrations. By providing the individual probes of a group of probes in different concentrations, it is possible to generate a "weighted" signal, where some biological molecules of the first group of biological molecules may contribute more strongly to the combined detectable signal, if their respective probe is present in a higher concentration than other probes. This may be achieved for example in a qPCR reaction, wherein the first group of probes is a mix of primers for housekeeping genes, wherein the primers for some genes are present at a higher concentration than primers for other genes. Or for microarrays, the number or amount of oligonucleotides of one gene in one array spot is different to the number or amount of oligonucleotides of another gene in the same shared array spot.

According to yet another different aspect of the present invention a biological molecule to be analyzed is selected from the group consisting of protein, peptide and polypeptide. In this case, the expression of genes can be analyzed at a protein-level. The first and the second probe from the first group of probes used for such an assay may be antibodies, for example. For this type of assay an ELISA or RIA format maybe used. Here different antibodies or capture molecules for the respective protein are coupled to the same label and mixed in one reaction.

The method of the present invention can be performed in single reaction vessel type formats (e.g. an Eppendorf cup), wherein the first group of probes using a first label and possibly a second group of probes using a second label or a further probe for an individual gene of interest using a further label is added into the single reaction vessel along with the sample.

The method of the present invention can readily be adapted to microtiter plate formats, e.g. by precoating wells with the first group of probes using a first label and possibly a second group of probes using a second label or a further probe for an individual gene of interest using a further label.

The common inventive concept of different aspects of the present invention is the joint analysis of a group of metagenes in a single reaction, in other words, the multiplexing of individual genes in a single reaction as opposed to analyzing each gene in an individual reaction, which may referred to as "Metaplexing". The here described method of metaplexing leads to more reliable results in less steps, consumes less sample material and expensive reagents and thus reduces the costs.

When using the method according to the present invention for the determination of an expression level of housekeeping genes to be used as internal control, it was surprisingly found that the combined determination for a plurality of two or more housekeeping genes gave a very stable and reliable signal which was more stable and constant than using a computed average of the respective expression levels determined in separate assays.

The invention is now described in more detail in connection with the Figures, in which:
Figure 1 shows CT values of 42 patients measured for individual housekeeping genes according to the prior art;
Figure 2 shows CT values of 42 patients measured for metagenes according to the present invention; and
Figure 3 shows the selection of metagenes or individual genes as controls by an algorithm.

Many housekeeping genes like GAPDH, RPL37A, ACTB/G, and others are highly expressed in human tissues so that sensitivity is not a problem for quantitative PCR. But what is needed, is a bundle of housekeeping genes that give a stable signal for all samples to be analyzed. The here presented method of metaplexing reduces the number of outliers that are seen in normal qPCR reactions from patient samples. Metaplexing, e.g. qPCR of a bundle of housekeeping genes smoothes the normalizing reference point for genes of interest of a respective patient to a stable signal.

In case of RNA analysis to determine an expression level, RNA may be isolated by any known suitable method, e.g. using commercially available RNA isolation kits.

According to a preferred embodiment of the present invention, after lysis the samples are treated with silica-coated magnetic particles and a chaotropic salt, in order to purify nucleic acids contained in the sample for further analysis. This method of RNA isolation has been shown to yield satisfactory results even when RNA is extracted from fixed tissue samples. This method which allows successful purification of mRNA out of fixed tissue samples is disclosed in WO 030058649, WO 2006136314A1 and DE10201084A1, the content of which is incorporated herein by reference.

This RNA extraction method comprises the use of magnetic particles coated with silica (SiO₂). These highly pure magnetic particles coated with silica (silicon dioxide) are used for isolating nucleic acids, including DNA and RNA from cell and tissue samples, the particles, the particles may be retrieved from a sample matrix or sample solution by use of a magnetic field. These particles are particularly well-suited for the automatic purification of nucleic acids, mostly from biological samples for the purpose of detecting nucleic acids.

The selective binding of nucleic acids to the surface of these particles is due to the affinity of negatively charged nucleic acids to silica containing media in the presence of chaotropic salt like guanidine isothiocyanate. The binding properties are for example described in EP 819696.

This approach is particularly useful for the purification of mRNA from formalin and/or paraffin fixed tissue samples. In contrast to most other approaches which may result in small fragments that are not well suited for later gene expression analysis by PCR and/or hybridization technologies, this approach creates mRNA fragments which are large enough to allow such analysis.

This approach allows a highly-specific determination of gene expression levels with one of the above-mentioned methods, in particular hybridization based methods, PCR-based methods and/or array-based methods even in formalin and/or paraffinfixed tissue samples and is therefore highly efficient in the context of the present invention, because it allows the use of fixed tissue samples which often are readily available in tissue banks and connected to clinical data bases, e.g. for follow up studies to allow retrospective analysis.

Measurement of the expression level of e.g. housekeeping genes can be performed on the mRNA level by any suitable method, e.g. qPCR or gene expression array platforms, including commercially available platforms, such as Affymetrix, Illumina, Luminex, planar wave guide, electrochemical microarray chips, microarray chips with optical, magnetic, electrochemical or gravimetric detection systems and others or on a protein level by immunochemical techniques such as ELISA.

The term "planar waveguide" relates to methods, wherein the presence or amount of a target molecule is determined by using a planar wave guide detector which emits an evanescent field in order to detect the binding of a labeled target molecule, such as e.g. described in WO200113096-A1, the content of which is incorporated by reference herein.

The term "optical detection" relates to methods which detect the presence or amount of a target molecule through a change in optical properties, e.g. fluorescence, absorption, reflectance, chemiluminescence, as is well known in the art.

The term "magnetic detection" relates to methods which detect the presence or amount of a target molecule or label through a change in magnetic properties, e.g. through the use of XMR sensors, GMR sensors or the like.

The term "electrochemical detection" relates to methods which make use of an electrode system to which molecules, particularly biomolecules like proteins, nucleic acids, antigens, antibodies and the like, bind under creation of a detectable signal. Such methods are for example disclosed in WO0242759 WO200241992 and WO2002097413, the content of which is incorporated by reference herein. These detectors comprise a substrate with a planar surface and electrical detectors which may adopt, for example, the shape of interdigital electrodes or a two dimensional electrode array. These electrodes carry probe molecules, e.g. nucleic acid probes, capable of binding specifically to target molecules, e.g. target nucleic acid molecules and allowing a subsequent electrochemical detection of bound target nucleic acids.

The term "gravimetric detection" relates to methods which make use of a system that is able to detect changes in mass which occur e.g. when a probe binds its target.

### Example 1:

In experiments to identify the best housekeeping gene mixtures to be used as normalization genes for breast cancer prognosis or prediction, it was surprisingly found that mixtures of primers and probes of different housekeepers perform similar to separate measurements of the single genes by qPCR. For example, primers and probes where mixed for four housekeeping genes into one PCR well (ACTG1, CALM2, OAZ1 und PPIA). With one fourth of the concentration of each single reaction (1+1+1+1) it was surprisingly found that the cycle threshold value of the mean of single reactions was nearly identically to the measured cycle threshold value of the mixed reaction. In a second experiment two more housekeeping genes (GAPDH and RPL37A) were added and gave similar results for up to six genes in a metaplexing reaction.

Single genes in the mixture can also be weighted in the metaplexing reaction by raising the relative amount of primers and probes compared to other genes in the mixture. Mastermix, enzymes, MgSO4 are present in standard concentrations or are adjusted for optimized primer and probe performances.

### Example 2:

In a representative example, quantitative reverse transcriptase PCR was performed according to the following protocol:

| Primer/Probe Mix: | |
|---|---|
| 50 µl | 100 µM Stock Solution Forward Primer |
| 50 µl | 100 µM Stock Solution Reverse Primer |
| 25 µl | 100 µM Stock Solution Taq Man Probe bring to 1000 µl with water |

10 µL Primer/Probe Mix (1:10) are lyophilized, 2.5 h RT

| RT-PCR Assay set-up for 1 well: | |
|---|---|
| 3.1 | µl Water |
| 5.2 | µl RT qPCR MasterMix (Invitrogen) & Rox |
| 0.5 | µL MgSO4 (to 5.5 mM final concentration) |
| 1 | µl Primer/Probe Mix dried |
| 0.2 | µl RT/Taq Mx (-RT: 0.08 µL Taq) |
| 1 | µl RNA (1:2) |

| Thermal Profile: | |
|---|---|
| 50 °C | 30 Min * |
| 8 °C | ca. 20 Min * |
| 95 °C | 2 Min |
| 95 °C | 15 Sec. |
| 60 °C | 30 Sec. |
| 40 cycles | |

Table 2 shows probe sequences used in qPCR (termed qPCR-probe), tables 3 and 4 show the respective forward and reverse primer sequences and table 5 shows the sequences of the obtained amplicons/PCR products. Sequences are identified by respective SEQ ID NOs.

**Table 2: qPCR-Probe Sequences**

| **SEQ ID** | | | | |
|---|---|---|---|---|
| **Gene** | **RefSeq or ID** | **Probe** | **NO:** | **FAM 5' Sequence 3' TAMRA** |
| ACTG1 | NM_001614 | R113 | 1 | AGGCCCCCCTGAACCCCAAG |
| PPIA | NM_021130 | R115 | 2 | TGGTTGGATGGCAAGCATGTGGTG |
| CALM2 | NM_001743 | R117 | 3 | TCGCGTCTCGGAAACCGGTAGC |
| OAZ1 | NM_004152 | BC268 | 4 | TGCTTCCACAAGAACCGCGAGGA |
| GAPD | NM_02046 | R15 | 5 | AAG GTG AAG GTC GGA GTC AAC GGA TTT G |
| RPL37A | NM_000998 | R16 | 6 | TGGCTGGCGGTGCCTGGA |

**Table3: Forward Primer Sequences**

| **Gene** | **5' Primer** | **SEQ ID NO:** | **Sequence 3'** |
|---|---|---|---|
| ACTG1 | R113-for | 7 | CTGGCACCACACCTTCTACAAC |
| PPIA | R115-for | 8 | TTTCATCTGCACTGCCAAGACT |
| CALM2 | R117-for | 9 | GAGCGAGCTGAGTGGTTGTG |
| OAZ1 | BC268-for | 10 | CGAGCCGACCATGTCTTCAT |
| GAPD | R15-for | 11 | GCC AGC CGA GCC ACA TC |
| RPL37A | R16-for | 12 | TGTGGTTCCTGCATGAAGACA |

**Table 4: Reverse Primer Sequences**

| **Gene** | **3' Primer** | **SEQ ID NO:** | **5' Seqence 3'** |
|---|---|---|---|
| ACTG 1 | 113-rev | 13 | CAAACATAATCTGAGTCATCTTCTCTCTGT |
| PPIA | R115-rev | 14 | TATTCATGCCTTCTTTCACTTTGC |
| CALM2 | R117-rev | 15 | AGTCAGTTGGTCAGCCATGCT |
| OAZ1 | BC268-rev | 16 | AAGCCCAAAAAGCTGAAGGTT |
| GAPD | R15rev | 17 | CCA GGC GCC CAA TAC G |
| RPL37A | R16rev | 18 | GTGACAGCGGAAGTGGTATTGTAC |

**Table 5: Gene amplicons**

| **SEQ ID** | | |
|---|---|---|
| **Gene** | **NO:** | **Amplicon** |
| ACTG1 | 19 | CTGGCACCACACCTTCTACAACGAGCTGCGCGTGGCCC CGGAGGAGCACCCAGTGCTGCTGACCGAGGCCCCCCTG AACCCCAAGGCCAACAGAGAGAAGATGACTCAGATTATG TTTG |
| PPIA | 20 | TTTCATCTGCACTGCCAAGACTGAGTGGTTGGATGGCAA GCATGTGGTGTTTGGCAAAGTGAAAGAAGGCATGAATA |
| CALM2 | 21 | GAGCGAGCTGAGTGGTTGTGTGGTCGCGTCTCGGAAAC CGGTAGCGCTTGCAGCATGGCTGACCAACTGACT |
| OAZ1 | 22 | CGAGCCGACCATGTCTTCATTTGCTTCCACAAGAACCGC GAGGACAGAGCCGCCTTGCTCCGAACCTTCAGCTTTTTG GGCTT |
| GAPD | 23 | GCCAGCCGAGCCACATCGCTCAGACACCATGGGGAAGG TGAAGGTCGGAGTCAACGGATTTGGTCGTATTGGGCGCC TGG |
| RPL37A | 24 | TGTGGTTCCTGCATGAAGACAGTGGCTGGCGGTGCCTG GACGTACAATACCACTTCCGCTGTCAC |

Table 6 shows the groups of probes used for measuring metagenes, which are designated as housekeeping gene mixtures. The mixture includes respective forward and reverse primer and qPCR-Probe.

**Table 6: Housekeeping Gene Mixtures, HKM:**

| **Housekeeper** **Mixture Name** | **Probes in Mixture** |
|---|---|
| HKM | R117 + R113 + BC268 + R115 |
| HKM-1 | R113 + BC268 + R115 |
| HKM-2 | R117 + BC268 + R115 |
| HKM-3 | R117 + R113 + R115 |
| HKM-4 | R117 + R113 + BC268 |
| HKMs | BC268 + R115 + R15 |
| HKMx | R117 + R113 + BC268 + R115 + R15+ R16 |
| HKM1246 | R117 + R113 + R115 + R16 |
| HKM1256 | R117 + R113 + R15+ R16 |
| HKM2345 | R113 + BC268 + R115 + R15 |
| HKM2456 | R113 + R115 + R15 + R16 |
| | |

Experiments showed that using the housekeeping gene mixtures (HKM) listed in table 6 produced consistently superior results as opposed to using single house keeping genes as internal reference. These HKM are groups of probes within the meaning of the present invention. In each HKM the same detectable label (a FAM/TAMRA labeled probe) was used, thus producing a combined detectable signal.

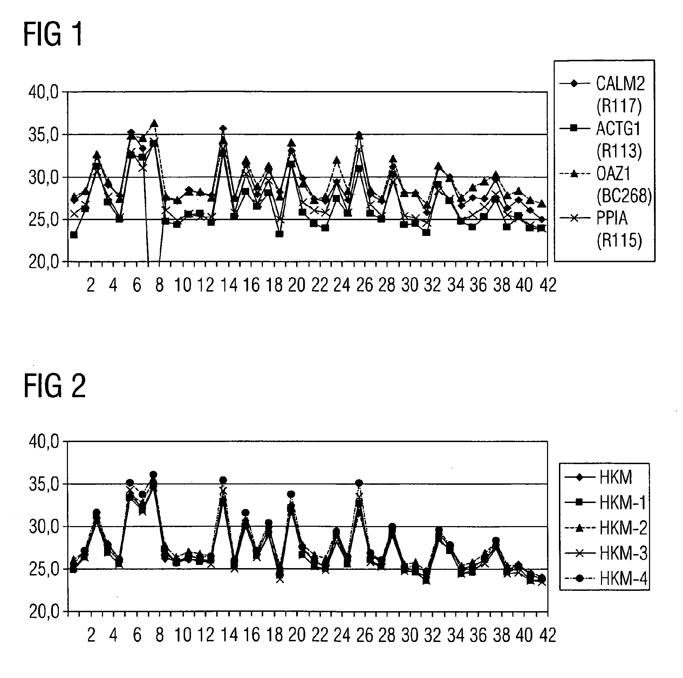

Fig. 1 shows CT (cycle threshold) values of 42 patients for the respective genes CALM2, ACTG1, OAZ1, PPIA. The 42 Patients are denoted on the x-axis, the CT values are denoted on the y-axis. It can be seen that there are considerable variations of the CT value of individual housekeeping genes tested for the same patient.

Fig. 2 shows CT values of the same 42 patients for the housekeeping gene mixtures HKM, HKM-1, HKM-2, HKM-3, and HKM-4. It can be seen, that the number of outliers is significantly reduced and that each housekeeping gene mixture produces a stable signal for each patient which is comparable to the signal of the other housekeeping gene mixtures.

### Example 3

To find an optimal housekeeping gene or mixture we propose a supervised approach that assigns each gene its optimal housekeeper or mixture.

In order to determine which house keeping gene or housekeeping gene mixture is best suited for statistical analysis and allows the best separation of patients with respect to expression of a gene of interest (GOI), separation was performed by using an algorithm, e.g. correlation coefficient, fisher score or t-test.

For each gene the housekeeper is selected in a way that, by subtracting its CT-value vector from the gene's CT-value vector, maximizes the information content of that gene with respect the output vector (here the output vector is a step function, '1' for positive patients and '0' for control patients). As a measure for the information content we use 3 criteria, correlation coefficient, Fisher score and t-test.

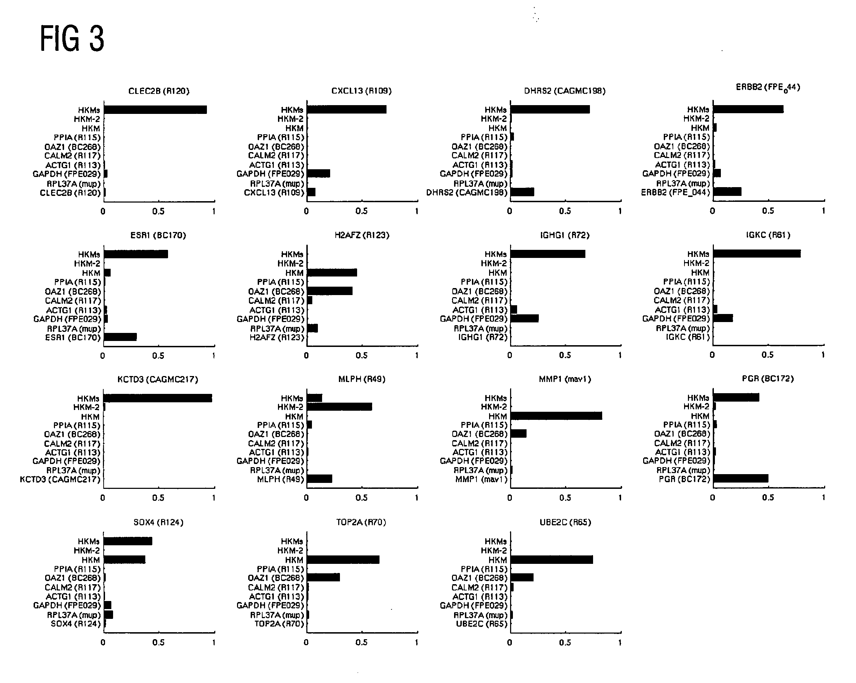

The approach was tested under a 100 times 10-fold cross validation setting to account for the stability of housekeeper-assignments. Figure 3 shows the relative frequency of housekeeping gene and mixture-assignments for each gene for 3 different criteria. As one can see, combined housekeeping gene mixtures, like HKMs, HKM-2 or HKM, are chosen much more frequently than individual housekeeping genes like RPL37A.

Fig. 3 shows how often a gene of interest uses one housekeeping gene or mixture for best separation of patients in the algorithm. Each panel in the Figure is designated by the name of the gene of interest for which separation was performed (e.g. CLEC2b, CXCL13, DHRS2, ErbB2 etc.). The y-axis of each panel lists the housekeeping gene or housekeeping gene mixture analyzed and the x-axis denotes the relative frequency with which a housekeeping gene or housekeeping gene mixture was chosen for best separation. The relative frequency is normalized (a relative frequency of 1.0 corresponding to 100%) so that the values of the bars in each panel add to 1.0. It can readily be seen that housekeeping gene mixtures were consistently preferred over individual housekeeping genes for best separation. The housekeeping gene mixture HKMs was most often chosen, e.g.for genes CLEC2b, CXCL13, DHRS2, ErbB2, ESR1, IgHG1, IgKC1, KCTD3, PGR). The housekeeping gene mixture HKM was chosen by MMP1 TOP2A and UBE2C, (all three genes also chose to a smaller extend OAZ1); HKM-2 was chosen by MLPH; HKMs + HKM were chosen by SOX4. The data shown in Fig. 3 was obtained using Fisher score as algorithm to choose housekeeping genes or mixtures for best separation of patients. Similar results are obtained using a correlation coefficient or t test (data not shown).

In the following some further examples of metaplexing are given, wherein not housekeeping genes are grouped for analysis, but other metagenes.

### Example 4

The method of the invention can be used for detection and expression analysis of cancer-specific nucleic acids in tissue samples, peripheral blood/sputum/stool/urine/liquor and other body fluids by screening.

Simultaneous detection can be performed according to the method of the present invention in a single reaction vessel (e.g. in an Eppendorf cup) using probes with specific sequences for different histological subtypes of one tumor type (e.g. for the analysis of lung cancer: adenocarcinoma vs. squamous cell carcinoma vs. small cell lung carcinoma; for the analysis of breast cancer: ductal carcinoma vs. lobular carcinoma vs. medullar carcinoma; for the analysis of gastrointestinal tumors: adenocarcinoma vs. stromal tumors vs. lymphoma vs. carcinoid).

Simultaneous detection can be performed according to the method of the present invention in a single reaction vessel using probes with specific sequences for different mutations of one tumor type or for detecting expression of different gene products (e.g. colon cancer: K-ras, APC, DCC, bcl-2, COX2) in tissue or body fluids.

Although the above examples use oligonucleotides as probes, the method of the present invention can also be performed at the level of protein expression, for example by using antibodies as probes. Such assays can be performed in an ELISA or RIA format using antibody-mixtures as a first group of probes using a first label and possibly for a second group of probes using a second label or for a further probe for an individual gene of interest using a further label.

The examples given above are for illustrating the present invention and are not intended to limit the scope of the present invention beyond what is defined in the claims.

## Claims

1. Method of analyzing a biological sample comprising the following steps:
a) providing a first group of probes comprising at least a first probe capable of specifically binding a first biological molecule belonging to a first group of biological molecules and a second probe capable of specifically binding a second biological different from the first biological molecule, but also belonging to the first group of biological molecules;
b) reacting said first probe and said second probe with said sample such as to enable formation of a binding complex of the probes with the respective biological molecule;
c) providing a first label yielding a detectable first signal upon binding of the first probe to the first biological molecule or upon binding of the second probe to the second biological molecule, wherein a combined detectable first signal results when one or both of first and second biological molecule are present in the sample; and
d) detecting said first signal to detect binding of probes with the respective biological molecule.

2. Method of claim 1 wherein the first group of probes comprises three or more differing probes for respective differing biological and a combined detectable first signal for three or more different biological molecules is obtained.

3. Method of claim 1 or 2 wherein a second group of probes different to the first group of probes and selected to be capable of binding biological molecules belonging to a second group of biological molecules is provided and a second label yielding a second signal different from the first signal of is provided, wherein the second signal results upon binding of one or more of the probes of the second group of probes to one or more corresponding biological molecules of the second group of biological molecules.

4. Method of any of claims 1 to 3, wherein a further probe specific to a further biological molecule is provided and a further label yielding a further signal different from the signal of the first and second label is provided, wherein the further signal results upon binding of the further probe to the further biological molecule.

5. Method of any of claims 1 to 4, wherein at least one of the first, second, and further signals is quantifiably detected.

6. Method of claim 5, wherein the signal quantification of the further signal is compared to the signal quantification of first or second signal.

7. Method of claim 6, wherein the signal quantification is correlated to a level of gene expression.

8. Method of claim any of claims 1 to 7, wherein the first group of biological molecules comprises mRNA of housekeeping genes.

9. Method of claim 8, wherein the first group of biological molecules comprises mRNA of housekeeping genes and the second group of biological molecules or the further biological molecule is linked to a specific phenotype or pathologic condition.

10. Method of any of claims 1 to 7 wherein the first or second group of biological molecules comprises biological molecules linked to a common phenotype or pathologic condition wherein a threshold signal strength for the first or second signal is determined and a signal strength either above or below said threshold signal indicates the presence of said phenotype or pathologic condition.

11. Method of claim 10, wherein in a subsequent step the presence or absence or amount of each member of the first group of biological molecules is then determined individually.

12. Method of any of claims 1 to 11 wherein at least one of the probes is an oligonucleotide comprising a sequence selected from the group consisting of SEQ ID NO:1 to SEQ ID NO:18.

13. Method of any of claims 1 to 12 wherein the first signal is quantifiably determined by quantitative PCR.

14. Method of any of claims 1 to 13, wherein the probes are provided on a microarray and wherein the first probe and second probe of the first group of probes are provided at the same array-position.

15. Method of any of claims 1 to 14 wherein the first probe and second probe of the first group of probes are provided at different concentrations.

16. Method of any of claims 1 to 15 wherein at least one biological molecule to be analyzed is selected from the group consisting of protein, peptide and polypeptide.

17. Method of claim 16, wherein the first and the second probe from the first group of probes are antibodies.

18. Kit for performing a method according to any of the preceding claims, comprising:
a) at least a first probe capable of specifically binding a first biological molecule belonging to a first group of biological molecules and a second probe capable of specifically binding a second biological different from the first biological molecule, but also belonging to the first group of biological molecules; and
b) a first label yielding a detectable first signal upon binding of the first probe to the first biological molecule or upon binding of the second probe to the second biological molecule, wherein a combined detectable first signal results when one or both of first and second biological molecule are present in the sample.

19. Kit of claim 18, wherein the first group of probes comprises three or more differing probes for respective differing biological molecules and a combined detectable signal for three or more different biological molecules is obtained.

20. Kit of claim 19 or 20 comprising a second group of probes different to the first group of probes and selected to be capable of binding biological molecules belonging to a second group of biological molecules and comprising a second label yielding a second signal different from the signal of the first signal, wherein the second signal results upon binding of one or more of the probes of the second group of probes to one or more corresponding biological molecules of the second group of biological molecules.

21. Kit of any of claims 18 to 20, comprising a further probe specific to a further biological molecule and comprising a further label yielding a further signal different from the first and second signal, wherein the further signal results upon binding of the further probe to the further biological molecule.

22. Kit of claim any of claims 18 to 21, wherein at least one of the probes is an oligonucleotide comprising a sequence selected from the group consisting of SEQ ID NO:1 to SEQ ID NO:18.

23. Kit of any of claims 18 to 22, wherein the first probe and second probe of the first group of probes are provided on the same position of a microarray.

24. Use of an oligonucleotide for performing the method according to any of claims 1 to 17, wherein said oligonucleotide comprises a sequence selected from the group consisting of SEQ ID NO:1 to SEQ ID NO:24.
